# EUROPEAN PATENT SPECIFICATION

(11) **EP 3 263 033 B1**
(45) Date of publication and mention of the grant of the patent: **11.12.2024**
(21) Application number: 17180269.7
(22) Date of filing: 26.01.2006
(51) Int. Cl.: A61B 5/145, A61M 5/142

(54) **AMBULATORY MEDICAL DEVICE AND METHOD OF COMMUNICATION BETWEEN MEDICAL DEVICES**
AMBULANTE MEDIZINISCHE VORRICHTUNG UND VERFAHREN ZUR KOMMUNIKATION ZWISCHEN MEDIZINISCHEN VORRICHTUNGEN
DISPOSITIF MÉDICAL AMBULATOIRE ET PROCÉDÉ DE COMMUNICATION ENTRE DES DISPOSITIFS MÉDICAUX

(30) Priority: 02.02.2005 EP 05002074
(43) Date of publication of application: 03.01.2018
(62) Divisional of application: 06703880.2
(73) Proprietor: F. Hoffmann-La Roche AG, 4070 Basel (CH); Roche Diabetes Care GmbH, 68305 Mannheim (DE)
(72) Inventor: ESSENPREIS, Matthias, 6330 Cham (CH); HAUETER, Ulrich, 3506 Grosshöchstetten (CH); BERNINI, Nicole, 4107 Ettingen (CH); KÄSER-FANKHAUSER, Sybille, 4542 Luterbach (CH); LA BASTIDE, Sebastiaan, Oro Valley, CA 85755 (US); MEYER-OLDEN, Gunnar, 26180 Rastede (DE); SCHOEMAKER, Michael, 68309 Mannheim (DE); HEATON, Kelly, Millwood VA 22646 (US); JECKELMANN, Joel, 1752 Villars-sur-Glane (CH)
(74) Representative: Rentsch Partner AG

(56) References cited:
- EP-A1- 1 338 295
- US-A1- 2004 068 230
- US-A1- 2004 167 464
- US-A1- 2004 225 338

## Description

### Technical field of the invention

The present invention relates to an ambulatory medical device and a method of communication for medical devices.

### Background Art

Ambulatory medical devices for the treatment of diabetes include e.g. extra corporal insulin pumps and blood glucose measuring devices such as e.g. hand held glucose meters. Insulin pumps allow a good control of blood glucose concentrations by continuously infusing a basic amount of insulin in a human body (basal insulin rate) and manually controlled additional "meal bolus" insulin quantities thereby reflecting the insulin secretion by the pancreas. Furthermore, the development of continuous glucose sensors will allow to measure in vivo glucose concentrations over the whole day. The measured glucose date can be used to adjust the diabetes therapy to individual needs.

In order to improve the treatment of diabetes it is important to provide means and methods to transfer data between medical devices in a quality assuring way It is therefore the aim of the present invention to provide a medical device allowing a controlled data transfer between medical devices and a method of controlled data transfer.

### Disclosure of the invention

In a first aspect, the present invention relates to a first medical device comprising a module for communication with at least one second medical device via a communication link, wherein the communication link is automatically activated by generating a value of a physiological parameter of an animal by the first medical device, wherein the communication link is established for a predetermined time span, wherein after expiration of the time span, the communication link is deactivated and no data exchange between the medical devices is anymore possible, wherein for further communication the communication link has to be activated by generating a further value of the physiological parameter.

The module for communication preferably is a telemetry system for wireless communication, preferably a telemetry system for RF communication.

Preferably the first medical device is selected from the group consisting of a remote control, a PDA, an analyte measuring device, preferably a glucose measuring device such as e.g. a hand held glucose meter, more preferably a strip based glucose meter.

The physiological parameter is preferably an analyte concentration of an animal.

In a preferred embodiment of the present invention the analyte concentration is a blood glucose concentration.

Preferably, the first medical device of the present invention comprises an electrochemical or photometric module for measuring blood glucose. Suitable medical devices are e.g. strip based glucose meters such as AccuChek Compact.

In a second aspect the present invention relates to a system of medical devices. Said system comprises a first medical device of the present invention as described in the previous section and at least a second medical device capable to communicate with said first medical device.

In a preferred embodiment the second medical device is selected from the group consisting of an extra corporal infusion pump, an implantable infusion pump, a pacesetter, an analyte or vital sign sensor, preferably a continuous analyte or vital sign sensor, more preferably a continuous glucose sensor.

In a further preferred embodiment the first medical device and the at least second medical device comprise a telemetry system for wireless communication, preferably a telemetry system for RF communication.

In a third aspect, the present invention relates to a method of communication between a first medical device and at least a second medical device wherein a communication link between said medical devices is automatically activated by generating a value of a physiological parameter of an animal by the first medical device, wherein the communication link is established for a predetermined time span, wherein after expiration of the time span, the communication link is deactivated and no data exchange between the medical devices is anymore possible, wherein for further communication the communication link has to be activated by generating a further value of the physiological parameter.

The physiological parameter is preferably selected from the group consisting of an analyte concentration of an animal, a rate, or an electrophysiological value. Preferably the analyte concentration is a blood glucose concentration.

In a further preferred embodiment, the first medical device is selected from the group consisting of a remote control, a PDA, an analyte measuring device, preferably a glucose measuring device, more preferably a strip based glucose meter.

The second medical device is preferably selected from the group consisting of an extra corporal infusion pump, an implantable infusion pump, a pacesetter, an analyte sensor, preferably a continuous analyte sensor, more preferably a continuous glucose sensor.

In a preferred embodiment, the communication between said medical devices is a wireless communication, preferably a RF communication.

In a further preferred embodiment, the first medical device receives data from the second medical device.

In a further preferred embodiment, the first medical device sends commands to the second medical device controlling at least partially the function of said device.

### Detailed description of the invention

In one aspect, the present invention relates to a novel method for controlling and/or enabling communication between medical devices, in particular medical sensory devices such as continuous glucose sensors and/or therapeutic devices such as insulin pumps and/or diagnostic medical devices such as glucose meters.

For example, the communication between a continuous glucose sensor applied to a human body and a blood glucose meter can only be established when a blood glucose measurement has been made in the blood glucose meter. The generation of the blood glucose value in the blood glucose meter enables and/or activates communication between the two devices for a specified time limit. During the time window data can be transferred from the sensor to the glucose meter and/or commands from the glucose meter can be sent to the sensor. After expiration of a time limit, communication between the two devices is deactivated. In order to establish a further communication, the communication link between the two devices has to be activated by generating a further blood glucose value in the glucose meter.

The term "generation of a value" as it is used herein encompasses any method or procedure for the determination of physiological parameters such as methods for the measurement of analyte values, in particular blood glucose values. Suitable methods for the determination of blood glucose values are e.g. electrochemical methods and photometric methods which are known to a person skilled in the art.

The dependence of the communication link between medical devices on an actual analyte value ensures the quality of the data transmitted from the medical sensory device and/or medical therapeutical device to the medical diagnostic device.

The data transferred from the sensor to the diagnostic device can be stored on the diagnostic device e.g. a glucose meter and be transferred to a third device such as a PDA or a computer, for further processing and/or analysis. By means of suitable software the data can be analyzed and used for e.g. bolus recommendation or adjustment of basal insulin rates for patients using an extra- or intra corporal insulin pump.

The communication link between the diagnostic device and a third device e.g. a computer, does not need activation by generation of a blood glucose value in said diagnostic device.

In a preferred embodiment, the present invention relates to a method of communication between a diagnostic medical device, preferably a blood glucose meter, and an infusion pump, preferably an extra corporal insulin pump. In this specific embodiment, the diagnostic medical device is used as a remote control to control the function of the infusion pump. After a blood glucose value has been generated in the blood glucose meter a communication link between meter and pump is enabled and/or activated for a defined time and commands can be transferred from the remote control i.e. the glucose meter, to the pump. It is as well possible to transfer data stored on the pump to the diagnostic device during the communication time window.

When the remote control of the infusion pump does not comprise a module for measuring blood glucose concentration, the communication between pump and remote control is activated by entering a current blood glucose value measured in a blood glucose meter in the remote control. The value can e.g. be entered using buttons of the remote control or can be transferred via a wireless or wired connection to the glucose meter. After the blood glucose value has been entered in the remote control, a communication link between remote control and infusion pump is established, preferably for a predetermined time span. After expiration of the time span, the communication is interrupted and no data exchange between the two devices is anymore possible. A further round of communication needs a new activation of the communication by entering a new, current blood glucose value in the remote control. The term remote control as used herein encompasses PDA, smart phones, a handy and pump specific remote controls.

The data transfer between the medical devices can be performed using known technologies and comprise wired connections as well as wireless communications. These technologies are known to a person skilled in the art. The preferred communication is a wireless communication, more preferably RF communication.

The data transfer between the devices can be encrypted in order to ensure that non-authorized third parties do not gain access to personal data of patients. Methods of encrypting data are known to a person skilled in the art.

In a further preferred embodiment, the communication between the medical devices is activated by a manipulation on the second medical device e.g. an insulin pump, such as pressing a button or lever, inserting a battery, using the touch screen, shaking, bumping or squeezing or the like.

In the following paragraph a preferred embodiment of the present invention is described.

The preferred system of medical devices comprises a continuous glucose sensor device which is placed on a human body in order to measure glucose value in interstitial fluid and a glucose meter. The sensor device comprises an electrochemical glucose sensor measuring the glucose concentration in the interstitial tissue in a predetermined manner. The sensor device further comprises an extra corporal part including processor means for controlling the sensor, memory for storing measured glucose values and a telemetry system for transmitting the data to a glucose meter, preferably a strip-based glucose meter. The glucose values stored on the sensor device are then transferred to a glucose meter via the telemetry system.

The communication between the two devices i.e. the wireless link, is established and/or activated by measuring the glucose concentration in a blood sample of a patient using the glucose meter, preferably a strip-based glucose meter. When a strip-based glucose meter is used, the patient inserts a strip in the glucose meter and puts a droplet of blood on the strip. The glucose meter measures and indicates on its display the blood glucose value. After measurement of the blood glucose value, the communication link can then be activated/established either by pressing a button on the glucose meter e.g. an activation button or by a direct electronic link to the processor controlling the glucose telemetry system such that the completion of the blood glucose measurement automatically activates the wireless link between the devices.

The communication link is then established and a data transfer between the medical devices is possible for a defined time span. After expiration of the defined time span the communication link is deactivated and no further data/commands can be transmitted between the medical devices. A new blood glucose measurement in the glucose meter is then necessary to open a new wireless link between the medical devices.

In a further aspect, the present invention relates to a method of data processing or data use. Said method is characterised in that data processing or data use is only possible after activation by a value of a physiological parameter. The method is preferably used for the processing of medical data such as data measured by a sensor applied on a human body.

In a preferred embodiment, the method is used for the processing of medical data which have been measured by a sensor device applied on a human being, preferably a continuous glucose sensor. The data are then e.g. transferred to a diagnostic medical device, preferably a blood glucose meter. The data are preferably transferred via a wireless link from the sensor device to the diagnostic device. In this case, each of the at least two medical devices comprises a telemetry system for wireless communication. The wireless communication can be bidirectional or unidirectional.

In a preferred embodiment, the processing of the data is only possible for a limited time span after activation by a value of a physiological parameter. When the defined time span for data processing has lapsed, no further data processing is possible without a new activation by a value of a physiological parameter.

In a further aspect, the present invention relates to a medical device comprising a module for data processing which is adapted to be activated by a value of a physiological parameter. Preferably said module comprises a microprocessor with a memory for storing data.

Said medical device is preferably a blood glucose meter. Preferably, the processing of data stored in the memory of the blood glucose meter is either activated by pressing a button on the glucose meter (an activation button) or by a direct electronic link to the processor controlling the data processing module/system such that the completion of the blood glucose measurement automatically activates data processing

The terms "data processing" or "data use" as they are used herein refer to any manipulation of data and comprise analysis of data, presentation of data, interpretation of data and indication of data.

## Claims

1. A first medical device comprising a module for communication with at least one second medical device via a communication link, wherein the communication link is automatically activated by generating a value of a physiological parameter of an animal by the first medical device, **characterized in that** the communication link is established for a predetermined time span, wherein after expiration of the time span, the communication link is deactivated and no data exchange between the medical devices is anymore possible, wherein for further communication the communication link has to be activated by generating a further value of the physiological parameter.

2. The first medical device of claim 1, wherein the module for communication is a telemetry system for wireless communication, preferably a telemetry system for RF communication.

3. The first medical device of claim 1 or 2, wherein the first medical device is selected from the group consisting of a remote control, a PDA, an analyte measuring device, preferably a glucose measuring device, more preferably a strip based glucose measuring device.

4. The first medical device of claims 1 to 3, wherein the physiological parameter is an analyte concentration of an animal.

5. The first medical device of claim 4, wherein the analyte concentration is a blood glucose concentration.

6. The first medical device of claim 5, wherein the first medical device comprises an electrochemical module for measuring blood glucose.

7. The first medical device of claim 5, wherein the first medical device comprises a photometric module for measuring blood glucose.

8. A system of medical devices comprising:
a) a first medical device according to claims 1 to 7 and
b) at least one second medical device capable to communicate with said first medical device.

9. The system of claim 8, wherein the at least one second medical device is selected from the group consisting of an extra corporal infusion pump, an implantable infusion pump, a pacesetter, an analyte sensor, preferably a continuous analyte sensor, more preferably a continuous glucose sensor.

10. The system of claim 9, wherein:
a) the first medical device is one of a remote control and a glucose measuring device; and
b) the at least one second medical device is selected from a group consisting of a continuous glucose sensor, an extra corporal infusion pump and an implantable infusion pump.

11. The system of one of claims 8 to 10, wherein the first medical device and the at least one second medical device comprise a telemetry system for wireless communication, preferably a telemetry system for RF communication.

12. A method of communication between a first medical device and at least one second medical device, wherein a communication link between said medical devices is automatically activated by generating a value of a physiological parameter of an animal by the first medical device, **characterized in that** the communication link is established for a predetermined time span, wherein after expiration of the time span, the communication link is deactivated and no data exchange between the medical devices is anymore possible, wherein for further communication the communication link has to be activated by generating a further value of the physiological parameter.

13. The method of one of claims 12 to 13, wherein the physiological parameter is selected from the group consisting of an analyte concentration of an animal, a heart rate, an electrophysiological value, preferably a blood glucose concentration.

14. The method of one of claims 12 to 13, wherein the first medical device is selected from the group consisting of a remote control, a PDA, an analyte measuring device, preferably a glucose measuring device, more preferably a strip-based glucose measuring device.

15. The method of one of claims 12 to 14, wherein the at least one second medical device is selected from the group consisting of an extra corporal infusion pump, an implantable infusion pump, a pacesetter, an analyte sensor, preferably a continuous analyte sensor, more preferably a continuous glucose sensor.

16. The method of claims 14 and 15, wherein communication is between the first medical device which is one of a remote control and a glucose measuring device and the at least one second medical device which is selected from a group consisting of a continuous glucose sensor, an extra corporal infusion pump and an implantable infusion pump.

17. The method of one of claims 12 to 16, wherein the communication between said medical devices is a wireless communication, preferably a RF communication.

18. The method of one of claims 12 to 17, wherein the first medical device receives data from the at least one second medical device.

19. The method of one of claims 12 to 18, wherein the first medical device sends commands to the at least one second medical device to control at least partially said at least one second device.

## Patentansprüche

1. Erste medizinische Vorrichtung, umfassend ein Modul zur Kommunikation mit mindestens einer zweiten medizinischen Vorrichtung über eine Kommunikationsverbindung, wobei die Kommunikationsverbindung durch Erzeugen eines Wertes eines physiologischen Parameters eines Tieres mittels der ersten medizinischen Vorrichtung automatisch aktiviert wird, **dadurch gekennzeichnet, dass** die Kommunikationsverbindung für eine vorbestimmte Zeitspanne hergestellt wird, wobei nach Ablauf der Zeitspanne die Kommunikationsverbindung deaktiviert wird und kein Datenaustausch zwischen den medizinischen Vorrichtungen mehr möglich ist, wobei die Kommunikationsverbindung für weitere Kommunikation durch Erzeugen eines weiteren Wertes des physiologischen Parameters aktiviert werden muss.

2. Erste medizinische Vorrichtung nach Anspruch 1, wobei das Modul zur Kommunikation ein Telemetriesystem für drahtlose Kommunikation, vorzugsweise ein Telemetriesystem für Hochfrequenzkommunikation, ist.

3. Erste medizinische Vorrichtung nach Anspruch 1 oder 2, wobei die erste medizinische Vorrichtung aus der Gruppe ausgewählt ist, die aus einer Fernsteuerung, einem PDA, einer Analytmessvorrichtung, vorzugsweise einer Glukosemessvorrichtung, stärker bevorzugt einer streifenbasierten Glukosemessvorrichtung, besteht.

4. Erste medizinische Vorrichtung nach den Ansprüchen 1 bis 3, wobei der physiologische Parameter eine Analytkonzentration eines Tieres ist.

5. Erste medizinische Vorrichtung nach Anspruch 4, wobei die Analytkonzentration eine Blutglukosekonzentration ist.

6. Erste medizinische Vorrichtung nach Anspruch 5, wobei die erste medizinische Vorrichtung ein elektrochemisches Modul zum Messen von Blutglukose umfasst.

7. Erste medizinische Vorrichtung nach Anspruch 5, wobei die erste medizinische Vorrichtung ein photometrisches Modul zum Messen von Blutglukose umfasst.

8. System aus medizinischen Vorrichtungen, umfassend:
a) eine erste medizinische Vorrichtung nach den Ansprüchen 1 bis 7 und
b) mindestens eine zweite medizinische Vorrichtung, die mit der ersten medizinischen Vorrichtung kommunizieren kann.

9. System nach Anspruch 8, wobei die mindestens eine zweite medizinische Vorrichtung aus der Gruppe ausgewählt ist, die aus einer extrakorporalen Infusionspumpe, einer implantierbaren Infusionspumpe, einem Schrittmacher, einem Analytsensor, vorzugsweise einem kontinuierlich messenden Analytsensor, stärker bevorzugt einem kontinuierlich messenden Glukosesensor, besteht.

10. System nach Anspruch 9, wobei:
a) die erste medizinische Vorrichtung eine von einer Fernsteuerung und einer Glukosemessvorrichtung ist; und
b) die mindestens eine zweite medizinische Vorrichtung aus einer Gruppe ausgewählt ist, die aus einem kontinuierlich messenden Glukosesensor, einer extrakorporalen Infusionspumpe und einer implantierbaren Infusionspumpe besteht.

11. System nach einem der Ansprüche 8 bis 10, wobei die erste medizinische Vorrichtung und die mindestens eine zweite medizinische Vorrichtung ein Telemetriesystem für drahtlose Kommunikation, vorzugsweise ein Telemetriesystem für Hochfrequenzkommunikation, umfassen.

12. Verfahren zur Kommunikation zwischen einer ersten medizinischen Vorrichtung und mindestens einer zweiten medizinischen Vorrichtung, wobei eine Kommunikationsverbindung zwischen den medizinischen Vorrichtungen durch Erzeugen eines Wertes eines physiologischen Parameters eines Tieres mittels der ersten medizinischen Vorrichtung automatisch aktiviert wird, **dadurch gekennzeichnet, dass** die Kommunikationsverbindung für eine vorbestimmte Zeitspanne hergestellt wird, wobei nach Ablauf der Zeitspanne die Kommunikationsverbindung deaktiviert wird und kein Datenaustausch zwischen den medizinischen Vorrichtungen mehr möglich ist, wobei die Kommunikationsverbindung für weitere Kommunikation durch Erzeugen eines weiteren Wertes des physiologischen Parameters aktiviert werden muss.

13. Verfahren nach einem der Ansprüche 12 bis 13, wobei der physiologische Parameter aus der Gruppe ausgewählt ist, die aus einer Analytkonzentration eines Tieres, einer Herzfrequenz, einem elektrophysiologischen Wert, vorzugsweise einer Blutglukosekonzentration, besteht.

14. Verfahren nach einem der Ansprüche 12 bis 13, wobei die erste medizinische Vorrichtung aus der Gruppe ausgewählt ist, die aus einer Fernsteuerung, einem PDA, einer Analytmessvorrichtung, vorzugsweise einer Glukosemessvorrichtung, stärker bevorzugt einer streifenbasierten Glukosemessvorrichtung, besteht.

15. Verfahren nach einem der Ansprüche 12 bis 14, wobei die mindestens eine zweite medizinische Vorrichtung aus der Gruppe ausgewählt ist, die aus einer extrakorporalen Infusionspumpe, einer implantierbaren Infusionspumpe, einem Schrittmacher, einem Analytsensor, vorzugsweise einem kontinuierlich messenden Analytsensor, stärker bevorzugt einem kontinuierlich messenden Glukosesensor, besteht.

16. Verfahren nach den Ansprüchen 14 und 15, wobei Kommunikation zwischen der ersten medizinischen Vorrichtung, die eine von einer Fernsteuerung und einer Glukosemessvorrichtung ist, und der mindestens einen zweiten medizinischen Vorrichtung, die aus einer Gruppe ausgewählt ist, die aus einem kontinuierlich messenden Glukosesensor, einer extrakorporalen Infusionspumpe und einer implantierbaren Infusionspumpe besteht, erfolgt.

17. Verfahren nach einem der Ansprüche 12 bis 16, wobei die Kommunikation zwischen den medizinischen Vorrichtungen eine drahtlose Kommunikation, vorzugsweise eine Hochfrequenzkommunikation ist.

18. Verfahren nach einem der Ansprüche 12 bis 17, wobei die erste medizinische Vorrichtung Daten von der mindestens einen zweiten medizinischen Vorrichtung empfängt.

19. Verfahren nach einem der Ansprüche 12 bis 18, wobei die erste medizinische Vorrichtung Befehle an die mindestens eine zweite medizinische Vorrichtung zum mindestens teilweisen Steuern der mindestens einen zweiten Vorrichtung sendet.

## Revendications

1. Premier dispositif médical comprenant un module pour une communication avec au moins un second dispositif médical par l'intermédiaire d'une liaison de communication, dans lequel la liaison de communication est automatiquement activée en générant une valeur d'un paramètre physiologique d'un animal par le premier dispositif médical, **caractérisé en ce que** la liaison de communication est établie pour un laps de temps prédéterminé, dans lequel après expiration du laps de temps, la liaison de communication est désactivée et aucun échange de données entre les dispositifs médicaux n'est plus possible, dans lequel pour une communication ultérieure la liaison de communication doit être activée en générant une autre valeur du paramètre physiologique.

2. Premier dispositif médical selon la revendication 1, dans lequel le module pour une communication est un système de télémétrie pour une communication sans fil, de préférence un système de télémétrie pour une communication RF.

3. Premier dispositif médical selon la revendication 1 ou 2, dans lequel le premier dispositif médical est choisi dans le groupe constitué par une télécommande, un PDA, un dispositif de mesure d'analyte, de préférence un dispositif de mesure de glucose, plus préférablement un dispositif de mesure de glucose par bandelette.

4. Premier dispositif médical selon les revendications 1 à 3, dans lequel le paramètre physiologique est une concentration d'analyte d'un animal.

5. Premier dispositif médical selon la revendication 4, dans lequel la concentration d'analyte est une concentration de glucose sanguin.

6. Premier dispositif médical selon la revendication 5, dans lequel le premier dispositif médical comprend un module électrochimique destiné à mesurer le glucose sanguin.

7. Premier dispositif médical selon la revendication 5, dans lequel le premier dispositif médical comprend un module photométrique destiné à mesurer le glucose sanguin.

8. Système de dispositifs médicaux comprenant :
a) un premier dispositif médical selon les revendications 1 à 7 et
b) au moins un deuxième dispositif médical capable de communiquer avec ledit premier dispositif médical.

9. Système selon la revendication 8, dans lequel l'au moins un deuxième dispositif médical est choisi dans le groupe constitué par une pompe à perfusion extracorporelle, une pompe à perfusion implantable, un stimulateur cardiaque, un capteur d'analyte, de préférence un capteur d'analyte en continu, plus préférablement un capteur de glucose en continu.

10. Système selon la revendication 9, dans lequel :
a) le premier dispositif médical est un parmi une télécommande et un dispositif de mesure de glucose ; et
b) l'au moins un deuxième dispositif médical est choisi dans un groupe constitué par un capteur de glucose en continu, une pompe à perfusion extracorporelle et une pompe à perfusion implantable.

11. Système selon l'une des revendications 8 à 10, dans lequel le premier dispositif médical et l'au moins un deuxième dispositif médical comprennent un système de télémétrie pour une communication sans fil, de préférence un système de télémétrie pour une communication RF.

12. Procédé de communication entre un premier dispositif médical et au moins un deuxième dispositif médical, dans lequel une liaison de communication entre lesdits dispositifs médicaux est automatiquement activée en générant une valeur d'un paramètre physiologique d'un animal par le premier dispositif médical, **caractérisé en ce que** la liaison de communication est établie pour un laps de temps prédéterminé, dans lequel après expiration du laps de temps, la liaison de communication est désactivée et aucun échange de données entre les dispositifs médicaux n'est plus possible, dans lequel pour une communication ultérieure la liaison de communication doit être activée en générant une autre valeur du paramètre physiologique.

13. Procédé selon l'une des revendications 12 à 13, dans lequel le paramètre physiologique est choisi dans le groupe constitué par une concentration d'analyte d'un animal, une fréquence cardiaque, une valeur électrophysiologique, de préférence une concentration de glucose sanguin.

14. Procédé selon l'une des revendications 12 à 13, dans lequel le premier dispositif médical est choisi dans le groupe constitué par une télécommande, un PDA, un dispositif de mesure d'analyte, de préférence un dispositif de mesure de glucose, plus préférablement un dispositif de mesure de glucose par bandelette.

15. Procédé selon l'une des revendications 12 à 14, dans lequel l'au moins un deuxième dispositif médical est choisi dans le groupe constitué par une pompe à perfusion extracorporelle, une pompe à perfusion implantable, un stimulateur cardiaque, un capteur d'analyte, de préférence un capteur d'analyte en continu, plus préférablement un capteur de glucose en continu.

16. Procédé selon les revendications 14 et 15, dans lequel la communication s'effectue entre le premier dispositif médical qui est un parmi une télécommande et un dispositif de mesure de glucose et l'au moins un deuxième dispositif médical qui est choisi dans un groupe constitué par un capteur de glucose en continu, une pompe à perfusion extracorporelle et une pompe à perfusion implantable.

17. Procédé selon l'une des revendications 12 à 16, dans lequel la communication entre lesdits dispositifs médicaux est une communication sans fil, de préférence une communication RF.

18. Procédé selon l'une des revendications 12 à 17, dans lequel le premier dispositif médical reçoit des données provenant de l'au moins un deuxième dispositif médical.

19. Procédé selon l'une des revendications 12 à 18, dans lequel le premier dispositif médical envoie des commandes à l'au moins un deuxième dispositif médical pour commander au moins partiellement ledit au moins un deuxième dispositif.
